# EUROPEAN PATENT APPLICATION

(11) **EP 2 722 039 A2**
(43) Date of publication of application: **23.04.2014**
(21) Application number: 13199253.9
(22) Date of filing: 12.11.2008
(51) Int. Cl.: A61K 9/48

(54) **Tri-molecular complexes and their use in drug delivery systems**

(30) Priority: 12.11.2007 US 987377 P
(62) Divisional of application: 08850366.9
(71) Applicant: Pharmaceutics International, Inc., Hunt Valley MD 21030 (US)
(72) Inventor: Hassan, Emadeldin, Towson, MD 21286 (US); GumudavelliI, Sridhar, Cockeysville, MD 21030 (US)
(74) Representative: Bridle, Andrew Barry

(57) **Abstract**

The invention provides a tri-molecular complex for use in enteric drug delivery systems comprising: (a) a hydrophilic, film-forming, water-soluble polymer; (b) an acid-insoluble polymer; and (c)an amino acid. The invention further provides a method or preparing such complexes.

## Description

### CROSS-REFERENCE TO RELATED APPLICATIONS

This patent application claims the benefit of U.S. Provisional Patent Application No. 60/987,377 filed November 12, 2007, which is incorporated by reference.

### BACKGROUND OF THE INVENTION

Enteric drug delivery is well known to protect the stomach from irritant medications or to protect the contents of a pharmaceutical product from gastric fluid so that the active ingredient is released at the desired time and location in the gastrointestinal tract. Various techniques for enteric drug delivery are described in the following references: U.S. Patent Nos. 5,330,759, 7,122,207, 4,462,839, 4,138,013, 7,094, 4,265,814, 5,330, 759, 6,685,962, 4,790,881 as well as in European Patent Publication EP 1184033 A1, International PCT Patent Publications WO 01/24780 A2, WO 98/50019 and WO 2004/030658 A1. Generally, these techniques are based on the coating of beads, tablets, and/or hard and soft capsules.

Soft gel capsules are particularly difficult to coat and have a higher potential for defects in the coat. Preferably, soft capsules that do not need a coating are used for enteric drug delivery. For example, WO 01/24780 A2 describes a soft gelatin capsule with a shell made of a mixture of gelatin and an enteric polymer in the form of free acid or alkali salts and an oily capsule fill material containing a benzimidazole derivative. Ammonium or alkali hydroxides are used to dissolve the enteric polymers. Similarly, WO 2004/030658 A1 discloses an enteric capsule wall for both hydrophilic and hydrophobic fill materials.

The techniques described in both WO 01/24789 and WO 2004/030658 A1 primarily depend on the use of alkali hydroxides to prepare the gel mass used in the manufacturing of the shell. The use of alkali hydroxides either required an additional step of neutralization or evaporation of excess alkali to avoid the degrading effect of the alkali on the enteric polymer as well as on gelatin itself. Other methods are further limited by the possibility of being incompatible with acid-labile medicines that may be affected by the free carboxylic group of the enteric polymer. A non-gelatin capsule shell is also described in US 4,790,881 using one or more hydrophilic polymer in presence of low water content (5% to 25% of the polymer weight) where said polymers are in a molecularly dispersed solution. The highly viscous gel masses required a special injection device and apply a complicated and expensive pressure molding technique.

Traditional macromolecular complexes are mainly ionic complexes and are typically rigid, non-elastic and, in many cases, irreversible. They are often made of two polymers typically having opposite charges. The resultant complexes are generally neutral, water insoluble precipitates. Because the precipitated complex is hydrophobic (due to neutralization of the electric charge and masking of the hydrophilic groups), the use of these bimolecular polymeric complexes are limited to controlled release preparations.

Highly elastic films cannot be produced directly from bimolecular polymeric complexes. Bimolecular alginate-chitosan complexes are used in manufacturing of controlled release microspheres. Bimolecular polymeric complexes can also be used to immobilize a water soluble polymer to other polymeric or non-polymeric surfaces, for example, immobilization with heparin by chitosan. In view of these limitations, bimolecular polymeric complexes are generally not suitable for use in enteric drug delivery.

Accordingly, the need remains for drug delivery systems that can be used in pharmaceutical dosage forms to control the location in the gastro-intestinal tract (e.g., the small intestine) where an active ingredient is released from the dosage form.

### BRIEF SUMMARY OF THE INVENTION

The invention provides a tri-molecular complex comprising: (a) a water-soluble polymer; (b) an acid-insoluble polymer; and (c) an amino acid. The tri-molecular complex of the invention is useful in drug delivery systems, preferably in enteric drug delivery systems, and more preferably in soft capsule dosage forms.

The invention further provides a tri-molecular complex comprising gelatin, acrylate-methacrylate copolymers, and arginine wherein the amount of acrylate-methacrylate copolymers is at least about 25% by weight of the amount of the acrylate-methacrylate copolymers and gelatin present.

In another embodiment, the invention also provides a tri-molecular complex comprising (a) a first water-soluble polymer, (b) a second water-soluble polymer, and (c) a bridging molecule, wherein the second water-soluble polymer is less water-soluble than the first water-soluble polymer. In this embodiment of the invention, the tri-molecular complex is suitable to control the release of therapeutic or diagnostic agents.

### DETAILED DESCRIPTION OF THE INVENTION

The present invention involves the use of a liaison (or bridging) molecule that is capable of interacting with two polymers to form a tri-molecular complex. Advantageously, tri-molecular complexes according to the invention are typically more hydrophilic, more elastic, and can remain in solution to cast elastic films or capsule shells more efficiently than bimolecular complexes. The mechanical strength of the tri-molecular complexes of the invention is greater than bimolecular complexes.

Without limiting the invention, it is believed that tri-molecular complexes possess stronger intermolecular forces of interactions due to enhanced flexibility of the complexes. The flexibility of tri-molecular complexes is greatly enhances by the liaison molecule that can penetrate into key functional groups on both polymeric molecules, building more ionic or hydrogen bonds leading to an improved structure. In contrast, bimolecular complexes may not have the same degree of interaction because of the stereo chemistry of the polymeric molecules that has limited molecular mobility, relative to that of small molecules.

Tri-molecular complexes of the present invention exhibit superior physical properties relating to strength and elasticity that make the complexes useful in drug delivery and, in particular, enteric drug delivery. For example, tri-molecular complexes can be used to generate more elastic, easier to fabricate barriers to control drug release. They can also exist in solution to provide synergistic improvement in viscosity of suspensions or gels at lower concentrations.

The water-soluble polymer useful in the tri-molecular complex of the invention can be any suitable hydrophilic, film-forming polymer. Suitable water-soluble polymers can be of natural or synthetic origin. Natural polymers can be proteins, for example. The polymers can be ionizable or non-ionizable. Ionizable polymers include polyanionic, polycationic, and amphoteric polymers. The water-soluble polymer can be a mixture of two or more suitable water-soluble polymers. Examples of suitable water-soluble polymers include gelatin, hydroxypropylmethylcellulose, hydroxypropylcellulose, and mixtures thereof. The gelatin may be supplied in any suitable form including, for example, 150 bloom, limed bone, type B. Preferably, the hydrophilic, film-forming polymer is gelatin.

The acid-insoluble polymer suitable for use in the tri-molecular complex can be any suitable polymer. Examples of acid-insoluble polymers include phthalate polymers such as cellulose acetate phthalate, alginic acid salts such as sodium alginate and potassium alginate, polymethacrylates such as acrylic acid/methacrylic acid copolymers (also referred to as acrylate-methacrylate copolymers), and mixtures thereof. The acid-insoluble polymer can be a mixture of two or more suitable polymers. Acrylic acid/methacrylic acid copolymers are supplied as a powder under the trade name Eudragit® by Evonik Industries or as a 30% aqueous dispersion under the trade name Eastacryl® by Eastman Chemical Company. Preferably, the acid-insoluble polymer is an acrylic acid/methacrylic acid copolymer.

The bridging molecule (also referred to as a liaison molecule) useful in the tri-molecular complex may be a low molecular weight bifunctional or multifunctional molecule or an oligomer or small polymer of carbohydrate, peptide, or other synthetic polymers. The bridging molecule may be a mixture of two or more components. Preferably, the molecular weight of the bridging molecule is not more than about 6000 daltons.

Preferably, the bridging molecule is an amino acid. The amino acid may have at least two functional groups capable of forming ionic or hydrogen bonds and reacts with the water-soluble polymer and acid-insoluble polymer to produce a tri-molecular complex suitable for use in enteric drug delivery systems. More preferably, the amino acid is arginine, lysine, or mixtures thereof, and most preferably the amino acid is arginine. The amino acid is present in any suitable amount to produce a tri-molecular complex.

In a preferred embodiment of the invention, the water-soluble polymer is gelatin, the acid-insoluble polymer is an acrylic acid/methacrylic acid copolymer and the amino acid is arginine.

In another embodiment of the invention, the tri-molecular complex comprises (a) a first water-soluble polymer, (b) a second water-soluble polymer, and (c) a bridging molecule, wherein the second water-soluble polymer is less water-soluble than the first water-soluble polymer. In this embodiment, the tri-molecular complex can be used to control the release of therapeutic or diagnostic agents.

The first water-soluble polymer may be the water-soluble polymer as herein. The second water-soluble polymer is less water-soluble than the first water-soluble polymer and may comprise any suitable polymer. Examples of polymers suitable for use as the second water-soluble polymer include cellulosic polymers such as ethyl cellulose, cellulose, carboxymethyl cellulose, and mixtures thereof. Other suitable polymers for use as the second-water soluble polymer include synthetic polymers such as acrylate-methacrylate polymers. Mixtures of cellulosic and synthetic polymers may be used.

The tri-molecular complex of the invention may optionally contain an aqueous solvent. Any suitable aqueous solvent can be used including, for example, water and alkaline solutions having a pH greater than 7.0. Preferably, the aqueous solvent is water.

A plasticizer may optionally be present in the tri-molecular complex. Any suitable plasticizer may be used in the invention including, for example, glycerin, glycerol, sorbitol, polyethylene glycol, citric acid, citric acid esters (e.g. triethylcitrate), and mixtures thereof. Preferably, the plasticizer is glycerin, triethylcitrate, or mixtures thereof. The plasticizer may be present in any suitable amount to permit the preparation of tri-molecular complex that can be used to form a drug delivery system including an enteric drug delivery system such as, for example, soft capsules.

The amount of acid-insoluble polymer present in the tri-molecular complex is generally at least about 25% of the total weight of the acid-insoluble polymer and water-soluble polymer. Preferably, the amount of acid-insoluble polymer is between about 25% and about 35% of the total weight of the acid-insoluble polymer and water-soluble polymer.

Where the acid-insoluble polymer is acrylate-methacrylate copolymers and the water-soluble polymer is gelatin, the amount of acrylate-methacrylate copolymers is generally at least about 25% of the total weight of the acrylate-methacrylate copolymers and gelatin. Preferably, the amount of acrylate-methacrylate copolymers is between about 25% and about 40% of the total weight of the acrylate-methacrylate copolymers acid-insoluble polymer and water-soluble polymer.

The tri-molecular complexes of the present invention are useful in drug delivery systems and, in particular, enteric drug delivery systems. For example, the tri-molecular complexes can be used in the preparation of both hard and soft capsules. Preferably, the tri-molecular complexes are used to prepare soft capsules. Capsules manufactured using such tri-molecular complexes resist the acidic environment of stomach, but yet dissolve in intestinal fluid.

In another aspect of the invention, the tri-molecular complex is used to produce clear films that preserve the water solubility and elasticity of the pharmaceutical dosage form. The clear films may be converted into soft or hard capsule dosage forms that encapsulate an active pharmaceutical ingredient.

The tri-molecular complex can be prepared in the form of a gel mass that is then used to produce finished dosage forms preferably soft capsules. The gel mass may be prepared by any suitable method. For example, the gel mass can be manufactured by mixing the water-soluble polymer, acid-insoluble polymer and amino acid in an aqueous solvent. The tri-molecular gel mass can then be converted into soft capsules by a suitable method including, for example, by using a rotary die encapsulation machine.

The gel mass of the tri-molecular complex of the invention can be prepared in the following steps: (1) suspending the acid-insoluble polymer in an acidic aqueous system with low buffer capacity; (2) mixing the water-soluble polymer with an amino acid; and (3) mixing the product of steps (1) and (2) to yield the tri-molecular complex. Alternatively, the gel mass can be prepared by mixing the water-soluble polymer, the acid-insoluble polymer, and amino acid in powder form followed by addition of water.

In another embodiment, the tri-molecular complexes can be used in drug delivery, specifically controlled release drug delivery systems. In such systems, the bridging molecule can be a small molecular weight bifunctional molecule or an oligomer of carbohydrate, peptide, or other synthetic polymers preferably having a molecular weight of not more than about 6000 daltons. The tri-molecular complexes may be used in any suitable controlled release drug delivery systems including, for example, matrix and film-coating systems.

The following examples further illustrate the invention but, of course, should not be construed as in any way limiting its scope.

### EXAMPLE 1

A tri-molecular complex of gelatin/acrylate-methacrylate copolymers/arginine (GAMA) was prepared having the following composition:

| **Components** | **Amount (grams)** |
|---|---|
| Gelatin, NF (150 bloom, Lime bone, Type B) | 300.0 |
| Glycerin | 180.0 |
| Eudragit L 100-55 | 150.0 |
| Arginine | 25.0 |
| Purified water, USP | 345.0 |

The tri-molecular complex of this example was prepared by dissolving arginine in water and mixing gelatin, glycerin and Eudragit L 100-55 in the arginine solution at 80°C for 2.0 hours resulting in a clear solution.

### EXAMPLE 2

The clear solution containing the tri-molecular complex according to Example 1 was cast in

to 0.8 - 0.9 mm thick films which were dried at ambient conditions to moisture content of 6-7%. The dried films remained intact after 2 hours incubation in 0.1N HCl solution at 37°C.

### EXAMPLE 3

A tri-molecular complex was prepared having the following composition:

| **Components** | **Amount (grams)** |
|---|---|
| Gelatin, NF (150 bloom , Lime bone, Type B) | 60.0 |
| Glycerin | 36.0 |
| Eudragit L 100-55 | 30.0 |
| Arginine | 15.0 |
| Purified water, USP | 100.0 |

The tri-molecular complex of this example was prepared as follows:
1. Gelatin and glycerin were dissolved in one part of water at 80°C for 60 minutes;
2. Arginine was dissolved in the other part of water;
3. Eudragit L 100-55 was dissolved in arginine solution by mixing at 50°C for 10 hours in a water bath; and
4. The Eudragit solution was then mixed with gelatin solution at 80°C resulting in a clear solution.

### EXAMPLE 4

The clear tri-molecular complex solution of Example 2 was cast into 0.8 - 0.9 mm thick films which were dried at ambient conditions to moisture content of 6-7%. Dried films remained intact after 2 hours incubation in 0.1N HCl solution at 37°C.

### EXAMPLE 5

This example describes the identification of a preferred concentration ratio of gelatin to acrylate-methacrylate copolymer to form a gelatin/acrylate-methacrylate/arginine (GAMA) complex.

A series of solutions containing Eudragit L 100 to gelatin ratios ranging from 1:10 to 1:2 were prepared as follows:
1. A 30% gelatin solution was obtained by dissolving gelatin (150 bloom, Type B) in a solution containing 5% w/v arginine at 80°C for one hour.
2. Eudragit L-100 solutions in 5% arginine solution in water were prepared at polymer concentrations of, 3%, 6%, 12% and 15% by heating Eudragit L-100 in arginine solution at 70°C for two hours.
3. Gelatin solution was mixed with an equal volume of individual Eudragit solutions.

The resulting clear solutions were subjected to viscosity measurement using a cone & plate Brookfield type DV III remoter (spindle 52, 10 rpm, 60°C). The binding forces (F_{b}) between gelatin, Eudragit and arginine were measured according to the method described by Hassan and Gallo (Journal of Pharmaceutical Research, 1990).

The binding forces (F_{b}) were calculated as follows:
ή complex = ∑ή ingredients + ή binding
ή binding = ή complex - ∑ή ingredients
F_{b} = ή binding x SR
wherein ή is viscosity in poise
SR is rate of shear (sec⁻¹)
F_{b} is force of binding in Dynes-cm².

The following table summarizes the results. Tri-molecular complexes were formed at Eudragit to gelatin weight ratios of 1:2.5 and 1:2 at a constant arginine concentration with positive calculated force of binding. No positive binding forces were identified at Eudragit to gelatin weight ratios of 1:10 and 1:5.

| Ratio of Eudragit L 100-55 to Gelatin | Viscosity of Eudragit L 100-55 | ή complex | ή binding | Binding Force of the complex, F_{b} (Dynes.cm⁻²) |
|---|---|---|---|---|
| 1:10 | 18 | 105 | -250 | -50 |
| 1:5 | 39 | 277 | -99 | -19.8 |
| 1:2.5 | 45 | 595 | 213 | 426 |
| 1:2 | 50 | 892 | 505 | 101 |
| Gelatin solution | 0 | 337 | 0 | 0 |

### EXAMPLE 6

This example describes a tri-molecular complex gel mass and a method for its preparation.

A 100 kg of tri-molecular complex was prepared having the following composition:

| **Components** | **Amount (kg)** |
|---|---|
| Gelatin, NF (150 bloom , Lime bone, Type B) | 28.0 |
| Glycerin | 16.0 |
| Eudragit L 100-55 | 14.0 |
| Triethylcitrate | 1.4 |
| Arginine | 7.0 |
| Purified water, USP | 41.0 |

The tri-molecular complex gel mass of this example was prepared by mixing gelatin, glycerin and half of the purified water at 80°C for 60 minutes. Eudragit L 100-55 solution was prepared by dissolving the Eudragit in the second half of water after dissolving arginine in it. A clear Eudragit solution was obtained after mixing for 10 hours at 50°C. Eudragit solution was then mixed with gelatin solution at 80°C.

### EXAMPLE 7

This example demonstrates a method of manufacturing soft capsules using a tri-molecular complex gel mass.

Size 11 oblong oil filled capsules were manufactured using a Bochang rotary die encapsulation machine. The tri-molecular complex gel mass was cast to 0.7, 0.8, 0.9 and 1.0 mm ribbon and filled with 600 mg Miglyol oil per capsule. Capsules were dried to a moisture content of 3-5% and sustained 0.1N HCl at 37°C for 2.0 hrs.

All references, including publications, patent applications, and patents, cited herein are hereby incorporated by reference to the same extent as if each reference were individually and specifically indicated to be incorporated by reference and were set forth in its entirety herein.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

## Claims

1. A tri-molecular complex for use in enteric drug delivery systems comprising:
(a) a hydrophilic, film-forming, water-soluble polymer;
(b) an acid-insoluble polymer; and
(c) an amino acid.

2. The tri-molecular complex according to claim 1, further comprising an aqueous solvent.

3. The tri-molecular complex according to claim 1, further comprising a plasticizer.

4. The tri-moleculear complex according to claim 1, wherein the amino acid is arginine.

5. The tri-molecular complex according to claim 1, wherein the water-soluble polymer is gelatin, the acid-insoluble polymer is acrylic acid-methacrylic acid copolymers, and the amino acid is arginine.

6. The tri-molecular complex according to claim 3, wherein the plasticizer is selected from the group consisting of glycerin, triethylcitrate, and mixtures thereof.

7. The tri-molecular complex according to claim 5, wherein the amount of acrylate-methacrylate copolymers is at least about 25% by weight of the total amount of acrylate-methacrylate copolymers and gelatin.

8. The tri-molecular complex according to claim 6, wherein the plasticizer is a mixture of glycerin and triethylcitrate.

9. The tri-molecular complex according to claim 5, further comprising glycerin.

10. A tri-molecular complex for use in controlling the release of a therapeutic or diagnostic agent comprising:
(a) a hydrophilic, film-forming, water-soluble polymer;
(b) a second water-soluble polymer; and
(c) a bridging molecule,
wherein the second water-soluble polymer is less water-soluble than the hydrophilic, film-forming, water-soluble polymer.

11. The tri-molecular complex according to claim 10, wherein the second water-soluble polymer is a cellulosic polymer selected from the group consisting of a ethyl cellulose, cellulose, carboxymethyl cellulose, and mixtures thereof.

12. The tri-molecular complex according to claim 10, wherein the second water-soluble polymer is an acrylate-methacrylate polymer.

13. A method of preparing a tri-molecular complex comprising the following steps: (1) suspending the acid-insoluble polymer in an acidic aqueous system with low buffer capacity; (2) mixing the water-soluble polymer with an amino acid; and (3) mixing the product of steps (1) and (2) to yield the tri-molecular complex.
